(19) Europäisches Patentamt
European Patent Office
Office européen des brevets

(11) EP 1 236 478 B1

(12) EUROPEAN PATENT SPECIFICATION

(45) Date of publication and mention of the grant of the patent:
**26.10.2005 Bulletin 2005/43**

(51) Int Cl.[7]: **A61L 29/16**, A61L 29/08, A61L 31/10, A61L 31/16

(21) Application number: **02251370.9**

(22) Date of filing: **27.02.2002**

(54) **Peroxisome proliferator-activated receptor gamma ligand eluting medical device**

Peroxisome proliferator-activated receptor gamma Liganden freisetzende medizinische Vorrichtung

Dispositif médical éluant des ligands de peroxisome proliferator-activated receptor gamma

(84) Designated Contracting States:
**AT BE CH CY DE DK ES FI FR GB GR IE IT LI LU MC NL PT SE TR**

(30) Priority: **27.02.2001 US 271898**

(43) Date of publication of application:
**04.09.2002 Bulletin 2002/36**

(60) Divisional application:
**05018140.3**

(73) Proprietor: **Medtronic Vascular, Inc.**
**Santa Rosa, CA 95403 (US)**

(72) Inventors:
- **Carlyle, Wenda**
  **Petaluma, California 94952 (US)**
- **Cheng, Peiwen**
  **Santa Rosa, California 95409 (US)**
- **Cafferata, Robert L**
  **Santa Rosa, California 95405 (US)**

(74) Representative: **Green, Mark Charles et al**
**Urquhart-Dykes & Lord LLP**
**30 Welbeck Street**
**London W1G 8ER (GB)**

(56) References cited:
**EP-A- 0 950 386**
**WO-A-01/62238**
**WO-A-99/48529**
**US-A- 5 866 595**
**US-A- 5 925 657**

- **LINCOFF A MICHAEL ET AL: "Sustained local delivery of dexamethasone by a novel intravascular eluting stent to prevent restenosis in the porcine coronary injury model." JOURNAL OF THE AMERICAN COLLEGE OF CARDIOLOGY, vol. 29, no. 4, 1997, pages 808-816, XP002202663 ISSN: 0735-1097**
- **BRIEGER DAVID ET AL: "Local drug delivery systems and prevention of restenosis." CARDIOVASCULAR RESEARCH, vol. 35, no. 3, 1997, pages 405-413, XP002202664 ISSN: 0008-6363**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 236 478 B1

## Description

### FIELD OF THE INVENTION

The present invention relates generally to compositions and corresponding methods for preventing and/or treating restenosis in patients in need thereof. Specifically, the present invention relates to *in situ* peroxisome proliferator-activated receptor gamma (PPARγ) agonist delivery. More specifically, the present invention relates to PPARγ agonist eluting medical devices. In one embodiment of the present invention the medical devices include, without limitation, stents, catheters, probes and vascular grafts.

### BACKGROUND OF THE INVENTION

Cardiovascular disease, specifically atherosclerosis, remains a leading cause of death in developed countries. Atherosclerosis is a multifactorial disease that results in a narrowing, or stenosis, of a vessel lumen. Briefly, pathologic inflammatory responses resulting from vascular endothelium injury causes monocytes and vascular smooth muscle cells (VSMCs) to migrate from the sub endothelium and into the arterial wall's intimal layer. There the VSMC proliferate and lay down an extracellular matrix causing vascular wall thickening and reduced vessel patency.

Cardiovascular disease caused by stenotic coronary arteries is commonly treated using either coronary artery by-pass graft (CABG) surgery or angioplasty. Angioplasty is a percutaneous procedure wherein a balloon catheter is inserted into the coronary artery and advanced until the vascular stenosis is reached. The balloon is then inflated restoring arterial patency. One angioplasty variation includes arterial stent deployment. Briefly, after arterial patency has been restored, the balloon is deflated and a vascular stent is inserted into the vessel lumen at the stenosis site. The catheter is then removed from the coronary artery and the deployed stent remains implanted to prevent the newly opened artery from constricting spontaneously. However, balloon catheterization and stent deployment can result in vascular injury ultimately leading to VSMC proliferation and neointimal formation within the previously opened artery. This biological process whereby a previously opened artery becomes re-occluded is referred to as restenosis.

Treating restenosis requires additional, generally more invasive, procedures including CABG in some cases. Consequently, methods for preventing restenosis, or treating incipient forms, are being aggressively pursued. One possible method for preventing restenosis is the administration of medicaments that block local invasion/activation of monocytes thus preventing the secretion of growth factors that may trigger VSMC proliferation and migration. Metabolic inhibitors such as antineoplastic agents are currently being investigated as potential anti-restenotic compounds. However, the toxicity associated with the systemic administration of metabolic inhibitors has recently stimulated research into *in situ,* site-specific drug delivery.

Anti-restenotic coated stents are one potential method of site-specific drug delivery. Once the coated stent is deployed, it releases the anti-restenotic agent directly into the tissue thus allowing for clinically effective drug concentrations to be achieved locally without subjecting the recipient to side effects associated with systemic drug delivery. Moreover, localized delivery of anti-restenotic drugs results in anti-proliferative drug concentrations directly at the treatment thus eliminating the need for specific cell targeting technologies.

Recently, it has been reported that peroxisome proliferator-activated receptor γ (PPARγ) agonists administered systemically can significantly reduce neointimal formation and VSMC proliferation at six-months after coronary stent implantation in patients with non-insulin dependent diabetes mellitus (Takagi, T. et al. 2000. J. Am. Col. Card. 36(5) 1529-1534). Peroxisome proliferator-activated receptor γ is a member of a nuclear receptor super family that is activated by ligands such as certain fatty acids, eicosanoids and insulin-sensitising thiazolidinediones including rosiglitazone, pioglitazone, troglitazone, ciglitazone and others. (Jiang C. et al. 1998. Nature. 391: 82-85.) PPARγ agonists prevent vascular smooth muscle cell proliferation, migration and monocyte activation by inhibiting cyclin-dependent kinases (Law, R. E. et al. 1993. J. Clin. Invest. 98(8): 1897-1905 and Wakino, S. et al. 2000. J. Biol. Chem. 275(29): 22435-22441 and Kintscher et al. 2000. Eur. J. Pharm. 401 (3) 259-270. United States Patent Number 5,925,657 (the '657 patent)), discloses methods for reducing or preventing cytokine associated inflammatory responses. In one theoretical embodiment, an implantable medical device is used to deploy PPAR agonists. However, the '657 patent does not disclose vascular implants coated with PPARγ agonists in anti-proliferative concentrations suitable for preventing restenosis. Consequently, PPARγ agonists may prove valuable in treating and/or preventing restenosis.

United States patent number 5,866,595 (the '595 patent) discloses treating vascular retenosis using, calcium antagonists. Specifically, the '595 patent discloses the systemic use of Ciglitazone to block PDGF-derived calcium channels. However, the '595 patent does not disclose using a vascular stent to achieve the site-specific delivery of PPAR agonists. The prior art also teaches the sustained local delivery of Dexamethasone using a vascular stent (see Lincoff, A.M. et al. 1997. Sustained Local Delivery of Dexamethasome by Novel Intravascular Stent to Prevent Restensosis in the Porcine Coronarv Injury Model, J. Am. Coll. Cardiol, 29:808-16. [hereinafter "Lincoff"]) However, Lincoff teaches preventing inflammation associated with stent composition and delivery procedures, not hyperproliferation of vacular

smooth muscle cells. In fact, the author notes at page 814 that 'nevertheless, dexamethasome at the doses administered locally in this experiment did not decrease neointimal hyperlasia in the procine artery after stent overexpansion iniury."

Troglitazone, and other thiazolidinediones, are novel insulin-sensitising agents that significantly reduce hyperinsulinemia and hyper-glycemia (Law, R. E. et al. 1993. J. Clin. Invest. 98(8): 1897-1905). However, systemic troglitazone blood levels required to treat non-insulin dependent diabetes have been associated with liver toxicity. Consequently, troglitazone has been withdrawn from the market as a treatment for type II diabetes. Moreover, the troglitazone plasma levels required to treat diabetes are the same as those needed to prevent restenosis systemically making it unlikely that thiazolidinediones will be useful as systemic anti-restenotics. Therefore, the development of an *in situ* thiazolidinediones delivery platform would represent a significant advance in the treatment and prevention of restenosis.

## SUMMARY OF THE INVENTION

The present invention provides an *in situ* drug delivery platform that can be used to administer anti-restenotic tissue levels of peroxisome proliferator-activated receptor gamma (PPARγ) agonist without systemic side effects. The medical device of the invention comprises a vascular implant having a controlled release coating comprising at least one PPARγ agonist wherein the at least one PPARγ agonist is delivered locally to a blood vessel wall in an amount sufficient to inhibit cellular proliferation without inducing systemic toxicity. In one embodiment of the present invention the drug delivery platform is a medical device including, without limitations, stents, catheters, probes and vascular grafts.

In another embodiment of the present invention, a vascular stent is coated with thiazolidinedione PPARγ agonists. The thiazolidinediones can be attached to the vascular stent's surface using any means that provide a drug-releasing platform. Coating methods include, but are not limited to precipitation, coacervation, and crystallization. The thiazolidinediones of the present invention can be bound covalently, ionically, or through other intramolecular interactions including without limitation hydrogen bonding and van der Waals forces.

In another embodiment of the present invention the thiazolidinediones are complexed with a suitable biocompatible polymer. The polymer-drug complex is then used to either form a controlled-release medical device, integrated into a preformed medical device or used to coat a medical device. The biocompatible polymer may be any non-thrombogenic material that does not cause a clinically relevant adverse response. Suitable examples include, but are not limited to polyvinyl pyrrolidone, polytetrafluoroethylene, poly-L-lactic acid, polycaprolactone, polyethylene glycol, polystyrene, acrylates, polyesters, epoxies, silicones, cellulose and many others including co-polymers and blends thereof. Other methods of achieving controlled drug release are contemplated as being part of the present invention.

In another embodiment of the present invention the thiazolidinedione is selected from the non-limiting group consisting of 5-(4-[2-(N-methyl-N-(2-pyridyl)amino)ethoxy]benzyl)-2,4-thiazolidinedione (rosiglitazone), (+)-5-[[4-[(3,4-dihydro-6-hydroxy-2,5,7,8-tetramethyl-2H-1-benzopyran-2-yl) methoxy]phenyl]methyl]-2,4-thiazolidinedione (troglitazone), 5-[p-[1-methylcyclohexyl)methoxyl]benzyl]-2,4-thiazolidinedione (ciglitazone), 5-[p-[2-(5-ethyl-2-pyridyl)ethoxy]benzyl]-2,4-thiazolidinedione (pioglitazone), 5-[p-[3-(5-methyl-2-phenyl-4-oxazolyl)propionyl]benzyl]-2,4-thiazolidinedione (darglitazone), 5-[[(2R)-2-benzyl-6-chromanyl]methyl]-2,4-thiazolidinedione (englitazone), derivatives and combinations thereof. Moreover, the thiazolidinediones of the present invention can be combined with other anti-restenotic compounds including cytotoxic, cytostatic, anti-metabolic and anti-inflammatory compounds.

In yet another embodiment of the present invention an anti-restenotic compound coated stent can be combined with the local delivery of the same or another anti-restenotic compound to achieve a synergistic effect at the medical device placement site. This is particularly beneficial in that non-toxic therapeutic levels of both the thiazolidinediones and other anti-restenotic therapeutic can be combined to achieve dose specific synergism.

Additional embodiments of the present invention will be apparent to those skilled in the art from the detailed disclosure that follows.

## BRIEF DESCRIPTION OF THE DRAWINGS

Figure 1a graphically depicts percent inhibition of Human Coronary Artery Smooth Muscle Cell (HCASMC) proliferation at various concentrations of Ciglitazone.

Figure 1b graphically depicts HCASMC cell counts at day 4 at various concentrations of Ciglitazone.

Figure 1c graphically depicts HCASMC cytotoxicity at various concentrations of Ciglitazone.

Figure 2a graphically depicts percent inhibition of Human Coronary Artery Endothelial Cell (HCAEC) proliferation at various concentrations of Ciglitazone.

Figure 2b graphically depicts HCAEC cell counts at day 4 at various concentrations of Ciglitazone.

Figure 2c graphically depicts HCAEC cytotoxicity at various concentrations of Ciglitazone.

Figure 3 graphically depicts HCASMC cell counts at day 4 at various concentrations of Rosiglitazone.

## DETAILED DESCRIPTION

In the detailed description and claims that follows the compounds used to prevent restenosis may be referred to herein or elsewhere as PARRy agonists, thiazolidinediones, anti-restenotics, anti-restenotic compounds, drugs, therapeutics, anti-proliferatives, cytostatic agents, cytotoxic agents, or anti-metabolic agents. Furthermore, in the description and claims that follow, their trade name, chemical names or common names may refer to specific compounds. All of these terms may be used interchangeably without distinction and are all considered to within the scope of the present invention.

The present invention includes novel compositions and methods for delivering peroxisome proliferator-activated receptor gamma (PPARγ) agonists directly to tissues susceptible to restenosis. Specifically, the present invention is directed at implantable medical devices that provide for the *in situ,* site-specific controlled release of ligands that bind to and activate PPARγ receptors. Once activated, PPARγ receptors inhibit vascular smooth muscle cell (VSMC) proliferation.

In one embodiment of the present invention medical devices are provided with a PPAR γ agonist such as, but not limited to thiazolidinediones. Examples of PPAR γ agonist used in accordance with the teachings of the present invention include, but are not limited to 5-(4-[2-(N-methyl-N-(2-pyridyl)amino) ethoxy]benzyl)-2,4-thiazolidinedione (rosiglitazone), (+)-5-[[4-[(3,4-dihydro-6-hydroxy-2,5,7,8-tetramethyl-2H-1-benzopyran-2-yl) methoxy]phenyl]methyl]-2,4-thiazolidinedione (troglitazone), 5-[p-[1-methylcyclohexyl) methoxyl]benzyl]-2,4-thiazolidinedione (ciglitazone), 5-[p-[2-(5-ethyl-2-pyridyl) ethoxy]benzyl]-2,4-thiazolidinedione (pioglitazone), 5-[p-[3-(5-methyl-2-phenyl-4-oxazolyl) propionyl]benzyl]-2,4-thiazolidinedione (darglitazone), and 5-[[(2R)-2-benzyl-6-chromanyl]methyl]-2,4-thiazolidinedione (englitazone) (collectively referred to herein after as thiazolidinediones). Moreover, the present inventors also consider derivatives and analogous of the thiazolidinediones within the scope of the present invention.

Thiazolinediones of the present invention share a common molecular backbone comprising the thiazolidinedione group:

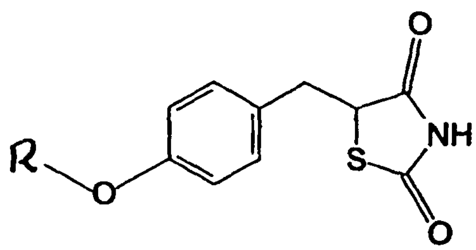

Wherein R is a branched or straight chain alkyl group of from 1 to 20 carbons, a branched or straight chain alkenyl group of from 1 to 20 carbons, a substituted or un-substituted aryl group, a substituted or un-substituted heterocyclic group, a substituted or un-substituted heteroaromatic group and combinations thereof.

Furthermore, derivatives made by modifying the thiazolidinedione group itself are also considered to be within the scope of the present invention. Therefore, it can be seen to those having ordinary skill in the art of medicinal chemistry that thiazolidinedione derivatives in addition to the specific thiazolidinediones recited herein are contemplated to be within the scope of the present invention.

The thiazolidinediones and other PPARγ agonists of the present invention are delivered, alone or in combination with synergistic and/or additive therapeutic agents, directly to the affected area using medical devices. Potentially synergistic and/or additive therapeutic agents may include drugs that impact a different aspect of the restenosis process such as antiplatelet, antimigratory or antifibrotic agents. Alternatively they may include drugs that also act as antiproliferatives and/or antiinflammatories but through a different mechanism than by binding to the PPARγ receptor. For example, and not intended as a limitation, synergistic combination considered to within the scope of the present invention include at least one thiazolidinedion and an antisense anti-c-myc oligonucleotide, at least one thiazolidinedion and rapamycin or analogues and derivatives thereof such as 40-0-(2-hydroxyethyl)-rapamycin, at least one thiazolidinedion and Exochelin, at least one thiazolidinedion and n-acetyl cysteine inhibitors, at least one thiazolidinedion and geldanamycin and other chaperone inhibitors, and so one.

The medical devices used in accordance with the teachings of the present invention may be permanent medical implants, temporary implants, or removable devices. For examples, and not intended as a limitation, the medical devices of the present invention may include, stents, catheters, micro-particles, probes and vascular grafts.

In one embodiment of the present invention stents are used as the drug delivery platform. The stents may be vascular stents, urethral stents, biliary stents, or stents intended for use in other ducts and organ lumens. Vascular stents may be used in peripheral, neurological or coronary applications. The stents may be rigid expandable stents or

pliable self expanding stents. Any biocompatible material may be used to fabricate the stents of the present invention including, without limitation, metals or polymers. The stents of the present invention may also be bioresorbable.

In one embodiment of the present invention vascular stents are implanted into coronary arteries immediately following angioplasty. However, one significant problem associated with stent implantation, specifically vascular stent deployment, is restenosis. Restenosis is a process whereby a previously opened lumen is re-occluded by VSMC proliferation. Therefore, it is an object of the present invention to provide stents that suppress or eliminate VSMC migration and proliferation and thereby reduce, and/or prevent restenosis.

In one embodiment of the present invention metallic vascular stents are coated with one or more anti-restenotic compound, specifically PPARγ agonists, more specifically the PPARγ agonists are thiazolidinediones. The thiazolidinediones may be dissolved or suspended in any carrier compound that provides a stable composition that does not react adversely with the device to be coated or inactivate the thiazolidinediones. The metallic stent is provided with a biologically active thiazolidinedione coating using any technique known to those skilled in the art of medical device manufacturing. Suitable non-limiting examples include impregnation, spraying, brushing, dipping and rolling. After the thiazolidinedione solution is applied to the stent it is dried leaving behind a stable thiazolidinedione delivering medical device. Drying techniques include, but are not limited to heated forced air, cooled forced air, vacuum drying or static evaporation. Moreover, the medical device, specifically a metallic vascular stent, can be fabricated having grooves or wells in its surface that serve as receptacles or reservoirs for the thiazolidinedione compositions of the present invention.

The preferred concentration of PPARγ agonist used in accordance with the teachings of the present invention can be determined using a titration process. Titration is accomplished by preparing a series of stent sets. Each stent set will be coated, or contain different dosages of the PPARγ agonist selected. The highest concentration used will be partially based on the known toxicology of the compound. The maximum amount of drug delivered by the stents made in accordance with the teaching of the present invention will fall below known toxic levels. Each stent set will be tested in vivo using the preferred animal model as described in Example 5 below. The dosage selected for further studies will be the minimum dose required to achieve the desired clinical outcome. In the case of the present invention, the desired clinical outcome is defined as the inhibition of vascular re-occlusion, or restenosis.

In another embodiment the thiazolidinediones are precipitated or crystallized on or within the stent. In yet another embodiment the thiazolidinediones are mixed with a suitable biocompatible polymer (bioerodable, bioresorbable or non-erodable) such as, but not limited to polyvinyl pyrrolidone, polytetrafluoroethylene, poly-L-lactic acid, polycaprolactone, polyethylene glycol, polystyrene, acrylates, polyesters, epoxies, silicones, cellulose and many others including blends thereof. The polymer-thiazolidinedione blend can then be used to produce a medical device such as, but not limited to stents, grafts, micro-particles, sutures and probes. Furthermore, the polymer-thiazolidinedione blend can be used to coat medical device surfaces. For example, and not intended as a limitation, the medical device can be immersed in the polymer-thiazolidinedione blend, or the polymer-thiazolidinedione blend can be sprayed, or brushed onto the medical device. In another embodiment, the polymer-thiazolidinedione blend can be used to fabricate fibers or strands that are embedded into the medical device or used to wrap the medical device.

Controlled *in situ* release of thiazolidinedione compositions of the present invention can be achieved using a variety of techniques known to those skilled in the art. For example, the polymer-thiazolidinedione blends of the present invention can be designed such that the polymer absorption rate dictates drug release. In one embodiment of the present invention a polycaprolactone-ciglitazone blend is prepared. A metallic vascular stent is then stably coated with polymer-blend wherein the stent coating has a thickness of between approximately 0.1 μm to approximately 100 μm. The polymer coating thickness determines the total amount of ciglitazone delivered and the polymer's absorption rate of polycaprolactone determines the administrate rate. Using this example, it is possible for one of ordinary skill in the part of polymer chemistry to design coatings having a wide range of dosages and administration rates. Furthermore, drug delivery rates and concentrations can also be controlled using non-polymer containing coatings and techniques known to persons skilled in the art of medicinal chemistry and medical device manufacturing,

The following examples are provided to more precisely define and enable the PPARγ agonist-eluting medical devices of the present invention. It is understood that there are numerous other embodiments and methods of using the present invention that will be apparent to those of ordinary skill in the art after having read and understood this specification and examples. These alternate embodiments are considered part of the present invention.

## EXAMPLES

### Providing a Metallic Surface with a PPARγ agonist-eluting Coating

The following Examples are intended to illustrate a non-limiting process for coating metallic stents with a PPARγ agonist and testing their anti-restenotic properties. One non-limiting example of a metallic stent suitable for use in accordance with the teachings of the present invention is the Medtronic/AVE S670™ 316L stainless steel coronary stent.

EXAMPLE 1

Metal Stent Cleaning Procedure

Stainless steel stents were placed a glass beaker and covered with reagent grade or better hexane. The beaker containing the hexane immersed stents was then placed into an ultrasonic water bath and treated for 15 minutes at a frequency of between approximately 25 to 50 KHz. Next the stents were removed from the hexane and the hexane was discarded. The stents were then immersed in reagent grade or better 2-propanol and vessel containing the stents and the 2-propanol was treated in an ultrasonic water bath as before. Following cleaning the stents with organic solvents, they were thoroughly washed with distilled water and thereafter immersed in 1.0 N sodium hydroxide solution and treated at in an ultrasonic water bath as before. Finally, the stents were removed from the sodium hydroxide, thoroughly rinsed in distilled water and then dried in a vacuum oven over night at 40°C.

After cooling the dried stents to room temperature in a desiccated environment they were weighed their weights were recorded.

EXAMPLE 2

Coating a Clean, Dried Stent Using a Drug/polymer System

250 mg of ciglitazone was carefully weighed and added to a small neck glass bottle containing 27.56 ml of tetrahydrofuran (THF). The ciglitazone-THF suspension was then thoroughly mixed until a clear solution is achieved.

Next 251.6 mg of polycaprolactone (PCL) was added to the ciglitazone-THF solution and mixed until the PCL dissolved forming a drug/polymer solution.

The cleaned, dried stents were coated using either spraying techniques or dipped into the drug/polymer solution. The stents were coated as necessary to achieve a final coating weight of between approximately 10 μg to 1 mg. Finally, the coated stents were dried in a vacuum oven at 50°C over night. The dried, coated stents were weighed and the weights recorded.

The concentration of drug loaded onto (into) the stents was determined based on the final coating weight. Final coating weight is calculated by subtracting the stent's pre-coating weight from the weight of the dried, coated stent.

EXAMPLE 3

Coating a Clean, Dried Stent Using a Sandwich-type Coating

A cleaned, dry stent was first coated with polyvinyl pyrrolidone (PVP) or another suitable polymer followed by a coating of ciglitazone. Finally, a second coating of PVP was provided to seal the stent thus creating a PVP-ciglitazone-PVP sandwich coated stent.

The Sandwich Coating Procedure:

100.2 mg of PVP was added to a 50 mL Erlenmeyer containing 12.5 ml of methanol. The flask was carefully mixed until all of the PVP is dissolved. In a separate clean, dry Erlenmeyer flask 252 mg of ciglitazone was added to 11 mL of THF and mixed until dissolved.

A clean, dried stent was then sprayed with PVP until a smooth confluent polymer layer was achieved. The stent was then dried in a vacuum oven at 50°C for 30 minutes.

Next the nine successive layers of the ciglitazone were applied to the polymer-coated stent. The stent was allowed to dry between each of the successive ciglitazone coats. After the final ciglitazone coating had dried, three successive coats of PVP were applied to the stent followed by drying the coated stent in a vacuum oven at 50°C over night. The dried, coated stent is weighed and its weight recorded.

The concentration of drug in the drug/polymer solution and the final amount of drug loaded onto the stent determine the final coating weight. Final coating weight is calculated by subtracting the stent's pre-coating weight from the weight of the dried, coated stent.

EXAMPLE 4

Coating a Clean, Dried Stent with Pure Drug

1.007 g of ciglitazone was carefully weighed and added to a small neck glass bottle containing 11.4 ml of ethyl alcohol (EtOH). The ciglitazone-EtOH suspension was then heated at 50°C for 15 minutes and then mixed until the ciglitazone was completely dissolved.

Next a clean, dried stent was mounted over the balloon portion of angioplasty balloon catheter assembly. The stent was then sprayed with, or in an alternative embodiment, dipped into, the ciglitazone-EtOH solution. The coated stent was dried in a vacuum oven at 50°C over night. The dried, coated stent was weighed and its weight recorded.

The concentration of drug loaded onto (into) the stents was determined based on the final coating weight. Final coating weight is calculated by subtracting the stent's pre-coating weight from the weight of the dried, coated stent.

EXAMPLE 5

In vivo Testing of a PPAR γ agonist-coated Vascular Stent in a Porcine Model

The ability of a PPAR γ agonist to reduce neointimal hyperplasia in response to intravascular stent placement in an acutely injured porcine coronary artery is demonstrated in the following example. Two controls and three treatment arms were used as outlined below:

1. Control Groups:

Six animals were used in each control group. The first control group tests the anti-restenotic effects of the clean, dried stent having neither polymer nor drug coatings. The second control group tests the anti-restenotic effects of polymer alone. Clean, dried stents having PCL coatings without drug were used in the second control group.

2. Experimental Treatment Groups

Three different stent configurations and two different drug dosages are evaluated for their anti-restenotic effects. Twelve animals are included in each group.

Group 1 stents are designated the fast release group and are comprised of 50 μg ciglitazone coated onto a bare stent without polymer in accordance with the teachings of the present invention.

Group 2, designated the slow-release group, uses stents coated with 50 μg of ciglitazone impregnated within a polymer at a ciglitazone to polymer ratio of 1:9 in accordance with the teachings of the present invention.

Group 3, designated the medium-release group, uses stents coated with 250 μg of ciglitazone impregnated within a polymer at a ciglitazone to polymer ratio of 1:1 in accordance with the teachings of the present invention.

The swine has emerged as the most appropriate model for the study of the endovascular devices. The anatomy and size of the coronary vessels are comparable to that of humans. Furthermore, the neointimal hyperplasia that occurs in response to vascular injury is similar to that seen clinically in humans. Results obtained in the swine animal model are considered predictive of clinical outcomes in humans. Consequently, regulatory agencies have deemed six-month data in the porcine sufficient to allow progression to human trials.

Non-atherosclerotic acutely injured RCA, LAD, and/or LCX arteries of the Farm Swine (or miniswine) are utilized in this study. Placement of coated and control stents is random by animal and by artery. The animals are handled and maintained in accordance with the requirements of the Laboratory Animal Welfare Act (P.L.89-544) and its 1970 (P.L. 91-579), 1976 (P.L. 94-279), and 1985 (P.L. 99-198) amendments. Compliance is accomplished by conforming to the standards in the Guide for the Care and the Use of Laboratory Animals, ILAR, National Academy Press, revised 1996. A veterinarian performs a physical examination on each animal during the pre-test period to ensure that only healthy pigs are used in this study.

A. Pre-Operative Procedures

The animals are monitored and observed 3 to 5 days prior to experimental use. The animals have their weight estimated at least 3 days prior to the procedure in order to provide appropriate drug dose adjustments for body weight. At least one day before stent placement, 650mg of aspirin is administered. Animals are fasted twelve hours prior to the procedure.

B. Anesthesia

Anesthesia is induced in the animal using intramuscular Telazol and Xylazine. Atropine is administered (20 μg/kg I.M.) to control respiratory and salivary secretions. Upon induction of light anesthesia, the subject animal is intubated. Isoflurane (0.1 to 5.0% to effect by inhalation) in oxygen is administered to maintain a surgical plane of anesthesia. Continuous electrocardiographic monitoring is performed. An I.V. catheter is placed in the ear vein in case it is necessary to replace lost blood volume. The level of anesthesia is monitored continuously by ECG and the animal's response to stimuli.

C. Catheterization and Stent Placement

Following induction of anesthesia, the surgical access site is shaved and scrubbed with chlorohexidine soap. An incision is made in the region of the right or left femoral (or carotid) artery and betadine solution is applied to the surgical site. An arterial sheath is introduced via an arterial stick or cutdown and the sheath is advanced into the artery. A guiding-catheter is placed into the sheath and advanced via a 0.035" guide wire as needed under fluoroscopic guidance into the ostium of the coronary arteries. An arterial blood sample is obtained for baseline blood gas, ACT and HCT. Heparin (200 units/kg) is administered as needed to achieve and maintain ACT ≥ 300 seconds. Arterial blood pressure, heart rate, and ECG are recorded.

After placement of the guide catheter into the ostium of the appropriate coronary artery, angiographic images of the vessels are obtained in at least two orthagonal views to identify the proper location for the deployment site. Quantitative coronary angiography (QCA) is performed and recorded. Nitroglycerin (200 µg I.C.) is administered prior to treatment and as needed to control arterial vasospasm. The delivery system is prepped by aspirating the balloon with negative pressure for five seconds and by flushing the guidewire lumen with heparinized saline solution.

Deployment, patency and positioning of stent are assessed by angiography and a TIMI score is recorded. Results are recorded on video and cine. Final lumen dimensions are measured with QCA and/or IVUS. These procedures are repeated until a device is implanted in each of the three major coronary arteries of the pig. After final implant, the animal is allowed to recover from anesthesia. Aspirin is administered at 325 mg p.o. qd until sacrifice.

D. Follow-up Procedures and Termination

After 28 days, the animals are anesthetized and a 6F arterial sheath is introduced and advanced. A 6F large lumen guiding-catheter (diagnostic guide) is placed into the sheath and advanced over a guide wire under fluoroscopic guidance into the coronary arteries. After placement of the guide catheter into the appropriate coronary ostium, angiographic images of the vessel are taken to evaluate the stented sites. At the end of the re-look procedure, the animal is euthanized with an overdose of Pentabarbitol I.V. and KCL I.V. The heart, kidneys, and liver are harvested and visually examined for any external or internal trauma. The organs are flushed with 1000 ml of lactated ringers at 100 mmHg and then flushed with 1000 ml of formalin at 100-120 mmHg. All organs are stored in labeled containers of formalin solution.

E. Histology and Pathology

The stented vessels will be X-rayed prior to histology processing. The stented segments are processed for routine histology, sectioned, and stained following standard histology lab protocols. Appropriate stains are applied in alternate fashion on serial sections through the length of the treated vessels.

F. Data Analysis and Statistics

1. QCA Measurement

Quantitative angiography is performed to measure the balloon size at peak inflation as well as vessel diameter pre- and post-stent placement and at the 28 day follow-up. The following data are measured or calculated from angiographic data:

Stent-to-artery-ratio
Minimum lumen diameter (MLD)
Distal and proximal reference lumen diameter

$$\text{Percent Stenosis} = (\text{Minimum lumen diameter} \div \text{reference lumen diameter}) \times 100$$

2. Histomorphometric analysis

Histologic measurements are made from sections from the native proximal and distal vessel and proximal, middle, and distal portions of the stent. A vessel injury score is calculated using the method described by Schwartz et al. (Schwartz RS et al. Restenosis and the proportional neointimal response to coronary artery injury: results in a porcine model. J Am Coll Cardiol 1992;19:267-74). The mean injury score for each arterial segment is calculated. Investigators scoring arterial segment and performing histopathology are "blinded" to the device type. The following

measurements are determined:

External elastic lamina (EEL) area
Internal elastic lamina (IEL) area
Luminal area
Adventitial area
Mean neointimal thickness
Mean injury score

3. The neointimal area and the % of in-stent restenosis are calculated as follows:

Neointimal area = (IEL-luminal area)
In-stent restenosis = [1-(luminal area ÷ IEL)] x 100.

A given treatment arm will be deemed beneficial if treatment results in a significant reduction in neointimal area and/or in-stent restenosis compared to both the bone stent control and the polymer-on control.

G. Surgical Supplies and Equipment

The following surgical supplies and equipment are required for the procedures described above:

1. Standard vascular access surgical tray
2. Non-ionic contrast solution
3. ACT machine and accessories
4. HCT machine and accessories (if applicable)
5. Respiratory and hemodynamic monitoring system
6. IPPB Ventilator, associated breathing circuits and Gas Anesthesia Machine
7. Blood gas analysis equipment
8. 0.035" HTF or Wholey modified J guidewire, 0.014" Guidewires
9. 6, 7, 8, and 9F introducer sheaths and guiding catheters (as applicable)
10. Cineangiography equipment with QCA capabilities
11. Ambulatory defibrillator
12. Standard angioplasty equipment and accessories
13. IVUS equipment (if applicable)
14. For radioactive labeled cell studies (if applicable):
15. Centrifuge
16. Aggregometer
17. Indium 111 oxime or other as specified
18. Automated Platelet Counter
19. Radiation Detection Device

<u>EXAMPLE 6</u>

<u>Inhibition of proliferation of Human Coronary Artery Smooth Muscle Cells by Ciglitazone</u>

A. Materials

1. Human coronary smooth muscles cells (HCASMC) were obtained from Clonetics, a division of Cambrex, Inc.
2. HCASMC basal Media, supplied by Clonetics and supplemented with fetal bovine serum, insulin, hFGF-B (human fibroblast growth factor) hEGF (human epidermal growth factor).
3. Ciglitazone Sigma Chemical Company catalogue number C-3974
4. Absolute Ethanol
5. Twenty-four well polystyrene tissue culture plates

B. Human coronary artery smooth muscle cells proliferation inhibition studies.

Human coronary smooth muscles cells (HCASMC) were seeded in 24 well polystyrene tissue culture plates at a density of 5 x$10^3$ cells per well. Two different feeding and reading strategies were employed. Strategy 1: Cells were plated in cell culture media containing various concentrations of Ciglitazone and incubated at 37° C for 48 hours. After the initial 48 hour incubation, the Ciglitazone containing plating media was changed and the cells were fed with drug free media and incubated for an additional 48 hours and then read.

Strategy 2: Cells were plated in cell culture media containing various concentrations of Ciglitazone and incubated at 37° C for 48 hours. After the initial 48 hour incubation, the Ciglitazone-containing plating media was changed and the cells were fed with Ciglitazone-containing media and incubated for an additional 48 hours and then read.

On day four cultures were analyzed to determine the proliferation inhibition effects of Ciglitazone. FIG. 1a graphically depicts the percent inhibition at Ciglitazone levels between 0.001 µg/mL to 50 µg/mL for both cell culture schemes. It can be seen from FIG 1a that significant HCASMC inhibition (>50% inhibition) begins at a dosage of 10 µg/mL and rises dramatically to nearly 100% at 50 µg/mL. FIG. 1b graphically depicts the same results in bar graph form based on cell counts.

C. Ciglitazone Cytotoxicity Testing

Ciglitazone cytotoxicity against HCASMCs was evaluated by seeding 24 well cell culture plates with 5.0 X $10^5$ HCASM cells/mL of cell culture media containing from 0.001 µg/mL to 10 µg/mL of Ciglitazone. Samples were taken after 24 hours and tested for lactate dehydrogenase (LDL) concentration using methods known to those having ordinary skill in the art. Elevated LDL levels indicates cytotoxicity. FIG. 1 c graphically depicts the cytotoxicity testing results. No cytotoxicity was detected at Ciglitazone concentrations that demonstrated significant anti-proliferative effects.

<u>EXAMPLE 7</u>

<u>Inhibition of proliferation of Human Coronary Artery Endothelial Cells by Ciglitazone</u>

A. Materials

1. Human coronary artery endothelial cells (HCAEC) were obtained from Clonetics, a division of Cambrex, Inc.
2. HCAEC basal Media, supplied by Clonetics and supplemented with fetal bovine serum, VEGF (vascular endothelial growth factor)hEGF heparin, ascorbic acid IGF (insulin growth factor) hydrocortisone
3. Ciglitazone Sigma Chemical Company catalogue number C-3974
4. Absolute Ethanol
5. Twenty-four well polystyrene tissue culture plates

B. Human coronary smooth muscles cells proliferation inhibition studies.

Human coronary artery endothelial cells (HCAEC) were seeded in 24 well polystyrene tissue culture plates at a density of 5 x$10^3$ cells per well. Two different feeding strategies and reading strategies were employed. Strategy 1: Cells were plated in cell culture media containing various concentrations of Ciglitazone and incubated at 37° C for 48 hours. After the initial 48 hour incubation, the Ciglitazone containing plating media was changed and the cells were fed with drug free media and incubated for an additional 48 hours and then read.

Strategy 2: Cells were plated in cell culture media containing various concentrations of Ciglitazone and incubated at 37° C for 48 hours. After the initial 48 hour incubation, the Ciglitazone-containing plating media was changed and the cells were fed with Ciglitazone-containing media and incubated for an additional 48 hours and then read.

On day four cultures were analyzed to determine the proliferation inhibition effects of Ciglitazone. FIG. 1a graphically depicts the percent inhibition at Ciglitazone levels between 0.001 µg/mL to 50 µg/mL for both cell culture schemes. It can be seen from FIG 2a that significant HCAEC inhibition (>50% inhibition) begins at a dosage of 5 µg/mL and rises dramatically to nearly 100% at 10 µg/mL. FIG. 2b graphically depicts the same results in bar graph form based on cell counts.

C. Ciglitazone Cytotoxicity Testing

Ciglitazone cytotoxicity against HCAECs was evaluated by seeding 24 well cell culture plates with 5.0 X $10^5$ HCAE cells/mL of cell culture media containing from 0.001 µg/mL to 10 µg/mL of Ciglitazone. Samples were taken

after 24 hours and tested for lactate dehydrogenase (LDL) concentration using methods known to those having ordinary skill in the art. Elevated LDL levels indicate cytotoxicity. FIG. 2c graphically depicts the cytotoxicity testing results. No cytotoxicity was detected at Ciglitazone concentrations that demonstrated significant anti-proliferative effects.

EXAMPLE 8

Inhibition of proliferation of Human Coronary Artery Smooth Muscle Cells by Rosiglitazone

A. Materials

1. Human coronary smooth muscles cells (HCASMC) were obtained from Clonetics, a division of Cambrex, Inc.
2. HCASMC basal Media, supplied by Clonetics and supplemented with fetal bovine serum, insulin, hFGF-B (human fibroblast growth factor) hEGF (human epidermal growth factor).
3. Rosiglitazone Sigma Chemical Company
4. Absolute Ethanol
5. Twenty-four well polystyrene tissue culture plates

B. Human coronary smooth muscles cells proliferation inhibition studies.

Human coronary smooth muscles cells (HCASMC) were seeded in 24 well polystyrene tissue culture plates at a density of 5 $x10^3$ cells per well. Two different feeding strategies and reading strategies were employed. Strategy 1: Cells were plated in cell culture media containing various concentrations of Rosiglitazone and incubated at 37° C for 48 hours. After the initial 48 hour incubation, the Rosiglitazone containing plating media was changed and the cells were fed with drug free media and incubated for an additional 48 hours and then read.
Strategy 2: Cells were plated in cell culture media containing various concentrations of Rosiglitazone and incubated at 37° C for 48 hours. After the initial 48 hour incubation, the Rosiglitazone-containing plating media was changed and the cells were fed with Rosiglitazone-containing media and incubated for an additional 48 hours and then read.
On day four cultures were analyzed to determine the proliferation inhibition effects of Rosiglitazone. FIG. 3 graphically depicts Rosiglitizone inhibition of HCASMC in bar graph form based on cell counts. Rosiglitazone levels between 0.001 µg/mL to 100 µg/mL for both cell culture schemes.
Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contain certain errors necessarily resulting from the standard deviation found in their respective testing measurements.
The terms "a" and "an" and "the" and similar referents used in the context of describing the invention (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided herein is intended merely to better illuminate the invention and does not pose a limitation on the scope of the invention otherwise claimed. No language in the specification should be construed as indicating any non-claimed element essential to the practice of the invention.
Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is herein deemed to contain the group as modified thus fulfilling the written description of all Markush

groups used in the appended claims.

Preferred embodiments of this invention are described herein, including the best mode known to the inventors for carrying out the invention. Of course, variations on those preferred embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Furthermore, numerous references have been made to patents and printed publications throughout this specification.

In closing, it is to be understood that the embodiments of the invention disclosed herein are illustrative of the principles of the present invention. Thus, by way of example, but not of limitation, alternative configurations of the present invention may be utilized in accordance with the teachings herein. Accordingly, the present invention is not limited to that precisely as shown and described.

## Claims

**1.** A medical device comprising a vascular implant having a controlled release coating comprising at least one PPARγ agonist wherein the at least one PPARγ agonist is delivered locally to a blood vessel wall in an amount sufficient to inhibit cellular proliferation without inducing systemic toxicity.

**2.** The medical device according to claim 1 wherein said PPARγ agonist is a thiazolidinedione.

**3.** The medical device according to claim 2 wherein said thiazolidinedione is selected from the group consisting of 5-(4-[2-(N-methyl-N-(2-pyridyl)amino) ethoxy]benzyl)-2,4-thiazolidinedione (rosiglitazone), (+)-5-[[4-[(3,4-dihydro-6-hydroxy-2,5,7,8-tetramethyl-2H-1-benzopyran-2-yl) methoxy]phenyl]methyl]-2,4-thiazolidinedione (troglitazone), 5-[p-[1-methylcyclohexyl) methoxyl]benzyl]-2,4-thiazolidinedione (ciglitazone), 5-[p-[2-(5-ethyl-2-pyridyl) ethoxy]benzyl]-2,4-thiazolidinedione (pioglitazone), 5-[p-[3-(5-methyl-2-phenyl-4-oxazolyl) propionyl]benzyl]-2,4-thiazolidinedione (darglitazone), 5-[[(2R)-2-benzyl-6-chromanyl]methyl]-2,4-thiazolidinedione (englitazone), derivatives thereof and combinations thereof.

**4.** The medical device according to claim 1 wherein said PPARγ agonist is ciglitazone.

**5.** The medical device according to any of claims 1, 2, 3 or 4 wherein the vascular implant further comprises stents, catheters, probes and vascular grafts.

**6.** The medical device according to claim 5 wherein said stent is a vascular stent or biliary stent.

**7.** The medical device according to claim 6 wherein said vascular stent is provided with a coating comprising at least one thiazolidinedione.

**8.** The medical device according to claim 7 wherein said thiazolidinedione is selected from the group consisting of rosiglitazone, pioglitazone, troglitazone, darglitazone, englitazone, ciglitazone, derivatives thereof and combinations thereof.

**9.** The medical device according to claim 8 wherein said coating further contains a biocompatible polymer selected from the group consisting of polyvinyl pyrrolidone, polytetrafluoroethylene, poly-L-lactic acid, polycaprolactone, polyethylene glycol, polystyrene, acrylates, polyesters and mixtures thereof.

**10.** The medical device according to claim 9 wherein said coating comprises:

betweent 50 μg to 250 μg of ciglitazone and polycaprolactone, wherein said ciglitazone and said polycaprolactone are in a ratio relative to each other of approximately 1 part ciglitazone to approximately between 1 to 9 parts polycaprolactone.

**11.** A method for producing a medical device comprising:

providing medical device to be coated;
compounding at least one thiazolidinedione selected from the group consisting of rosiglitazone, pioglitazone, troglitazone, darglitazone, englitazone, ciglitazone, derivatives thereof and combinations thereof with a carrier compound; and
coating said medical devices with said thiazolidinedione compounded with said carrier compound.

**12.** The method according to claim 11 wherein said medical device is a vascular stent.

**13.** The method according to claim 11 further wherein said carrier compound is a biocompatible polymer selected from the group consisting of polyvinyl pyrrolidone, polytetrafluoroethylene, poly-L-lactic acid, caprolactone, polyethylene glycol, polystyrene, acrylates, polyesters and mixtures thereof.

**14.** A medical device according to claims 8 to 10 comprising a stent having a coating, the coating comprising at least one thiazolidinedione selected from the group consisting of rosiglitazone, pioglitazone, troglitazone, darglitazone, englitazone, ciglitazone, derivatives thereof and combinations thereof; and
at least one additional therapeutic agent selected from the group consisting of antiplatelet agents, antimigratory agent, antifibrotic agents, antiproliferatives, antiinflammatories and combinations thereof providing that said additional therapeutic agent is not a PPARγ agonist.

**15.** The medical device according to claim 14 wherein said at least one additional therapeutic agent is selected from the group consisting of antisense oligonucleotides, rapamycin, analogues of rapamycin, exochelin, n-acetyl cysteine inhibitors, chaperone inhibitors and combinations thereof.

**16.** The medical device according to claim 15 wherein said antisense oligonucleotide is an anti-c-myc oligonucleotide.

**17.** The medical device according to claim 15 wherein said chaperone inhibitor is geldanamycin.

**18.** The medical device according to claim 15 wherein said rapamycin derivative is 40-0-(2-hydroxyethyl)-rapamycin.

## Patentansprüche

**1.** Medizinisches Gerät umfassend ein Gefäßimplantat, das eine Beschichtung zur gesteuerten Freisetzung aufweist, umfassend mindestens einen PPARγ-Agonisten, wobei der mindestens eine PPARγ-Agonist lokal an eine Blutgefäßwand in einer Menge geliefert wird, die ausreicht, um die Zellproliferation ohne Induzieren einer systemischen Toxizität zu hemmen.

**2.** Medizinisches Gerät nach Anspruch 1, wobei der PPARγ-Agonist ein Thiazolidindion ist.

**3.** Medizinisches Gerät nach Anspruch 2, wobei das Thiazolidindion aus der Gruppe ausgewählt wird bestehend aus 5-(4-[2-(N-Methyl-N-(2-pyridyl)amino)ethoxy]benzyl)-2,4-thiazolidindion (Rosiglitazon), (+)-5-[[4-[(3,4-Dihydro-6-hydroxy-2,5,7,8-tetramethyl-2H-1-benzopyran-2-yl)methoxy]phenyl]methyl]-2,4-thiazolidindion (Troglitazon), 5-[p-[1-Methylcyclohexyl)methoxyl] benzyl]-2,4-thiazolidindion (Ciglitazon), 5-[p-[2-(5-Ethyl-2-pyridyl)ethoxy]benzyl]-2,4-thiazolidindion (Pioglitazon), 5-[p-[3-(5-Methyl-2-phenyl-4-oxazolyl)propionyl]benzyl]-2,4-thiazolidindion (Darglitazon), 5-[[(2R)-2-Benzyl-6-chromanyl]methyl]-2,4-thiazolidindion (Englitazon), Derivaten derselben und Kombinationen derselben.

**4.** Medizinisches Gerät nach Anspruch 1, wobei der PPARγ-Agonist Ciglitazon ist.

**5.** Medizinisches Gerät nach einem der Ansprüche 1, 2, 3 oder 4, wobei das Gefäßimplantat des Weiteren Stents, Katheter, Sonden und Gefäßtransplantate umfasst.

**6.** Medizinisches Gerät nach Anspruch 5, wobei der Stent ein Gefäßstent oder ein Gallenstent ist.

**7.** Medizinisches Gerät nach Anspruch 6 , wobei der Gefäßstent mit einer Beschichtung ausgestattet ist, die mindestens ein Thiazolidindion umfasst.

**8.** Medizinisches Gerät nach Anspruch 7, wobei das Thiazolidindion aus der Gruppe ausgewählt wird bestehend aus

Rosiglitazon, Pioglitazon, Troglitazon, Darglitazon, Englitazon, Ciglitazon, Derivaten derselben und Kombinationen derselben.

**9.** Medizinisches Gerät nach Anspruch 8, wobei die Beschichtung des Weiteren ein biokompatibles Polymer enthält ausgewählt aus der Gruppe bestehend aus Polyvinylpyrrolidon, Polytetrafluorethylen, Poly-L-Milchsäure, Polycaprolacton, Polyethylenglykol, Polystyrol, Acrylaten, Polyestern und Mischungen derselben.

**10.** Medizinisches Gerät nach Anspruch 9, wobei die Beschichtung Folgendes umfasst:

50 µg bis 250 µg Ciglitazon und Polycaprolacton, wobei das Ciglitazon und das Polycaprolacton in einem Verhältnis zueinander von ca. 1 Teil Ciglitazon zu ca. 1 bis 9 Teilen Polycaprolacton stehen.

**11.** Methode für die Herstellung eines medizinischen Geräts umfassend:

das Bereitstellen des zu beschichtenden medizinischen Geräts;
das Compoundieren des mindestens einen Thiazolidindions ausgewählt aus der Gruppe bestehend aus Rosiglitazon, Pioglitazon, Troglitazon, Darglitazon, Englitazon, Ciglitazon, Derivaten derselben und Kombinationen derselben mit einer Trägerverbindung; und
das Beschichten des medizinischen Geräts mit der Thiazolidindionverbindung, die mit der Trägerverbindung compoundiert ist.

**12.** Methode nach Anspruch 11, wobei das medizinische Gerät ein Gefäßstent ist.

**13.** Methode nach Anspruch 11, wobei die Trägerverbindung des Weiteren ein biokompatibles Polymer ist ausgewählt aus der Gruppe bestehend aus Polyvinylpyrrolidon, Polytetrafluorethylen, Poly-L-Milchsäure, Caprolacton, Polyethylenglykol, Polystyrol, Acrylaten, Polyestern und Mischungen derselben.

**14.** Medizinisches Gerät nach Anspruch 8 bis 10, umfassend einen Stent, der eine Beschichtung aufweist, wobei die Beschichtung Folgendes umfasst:

mindestens ein Thiazolidindion ausgewählt aus der Gruppe bestehend aus Rosiglitazon, Pioglitazon, Troglitazon, Darglitazon, Englitazon, Ciglitazon, Derivaten derselben und Kombinationen derselben; und
mindestens ein zusätzliches therapeutisches Mittel ausgewählt aus der Gruppe bestehend aus Antithrombozyten, Antimigrationsmittel, Antifibrosemitteln, Antiproliferationsmitteln, entzündungshemmendes Mitteln und Kombinationen derselben, vorausgesetzt, dass das zusätzliche therapeutische Mittel kein PPARγ-Agonist ist.

**15.** Medizinisches Gerät nach Anspruch 14, wobei das mindestens eine zusätzliche therapeutische Mittel aus der Gruppe ausgewählt wird bestehend aus Antisenseoligonucleotiden, Rapamycin, Analogen von Rapamycin, Exochelin, n-Acetylcystein-Inhibitoren, Chaperoninhibitoren und Kombinationen derselben.

**16.** Medizinisches Gerät nach Anspruch 15, wobei das Antisenseoligonucleotid ein Anti-c-myc-Oligonucleotid ist.

**17.** Medizinisches Gerät nach Anspruch 15, wobei der Chaperoninhibitor Geldanamycin ist.

**18.** Medizinisches Gerät nach Anspruch 15, wobei das Rapamycinderivat 40-0-(2-Hydroxyethyl)-rapamycin ist.

## Revendications

**1.** Dispositif médical comprenant un implant vasculaire ayant un revêtement de séparation contrôlé comprenant au moins un agoniste PPARγ, dans lequel le au moins un agoniste PPARγ est délivré localement à une paroi de vaisseau sanguin dans une quantité suffisante pour inhiber la prolifération cellulaire sans induire une toxicité systémique.

**2.** Dispositif médical selon la revendication 1, dans lequel ledit agoniste PPARγ est une thiazolidinedione.

**3.** Dispositif médical selon la revendication 2, dans lequel ladite thiazolidinedione est sélectionnée parmi le groupe constitué de la 5-(4-[2-(N-méthyl-N-(2-pyridyl)amino)éthoxy]benzyl)-2,4-thiazolidinedione (rosiglitazone), de la

(+)-5-[[4-[(3,4-dihydro-6-hydroxy-2,5,7,8-tétraméthyl-2H-1-benzopyran-2-yl)méthoxy]phényl]méthyl]-2,4-thiazolidinedione (troglitazone), de la 5-[p-[1-méthylcyclohexyl)méthoxyl]benzyl]-2,4-thiazolidinedione (ciglitazone), de la 5-[p-[2-(5-éthyl-2-pyridyl)éthoxy]benzyl]-2,4-thiazolidinedione (pioglitazone), de la 5-[p-[3-(5-méthyl-2-phényl-4-oxazolyl)propionyl]benzyl]-2,4-thiazolidinedione (darglitazone), de la 5-[[(2R)-2-benzyl-6-chromanyl]méthyl]-2,4-thiazolidinedione (englitazone), des dérivés de ces derniers et des combinaisons de ces derniers.

**4.** Dispositif médical selon la revendication 1, dans lequel ledit agoniste PPARγ est la ciglitazone.

**5.** Dispositif médical selon l'une quelconque des revendications 1, 2, 3 ou 4, dans lequel l'implant vasculaire comprend en outre des stents, des cathéters, des sondes et des greffes vasculaires.

**6.** Dispositif médical selon la revendication 5, dans lequel ledit stent est un stent vasculaire ou un stent biliaire.

**7.** Dispositif médical selon la revendication 6, dans lequel ledit stent vasculaire est pourvu d'un revêtement comprenant au moins une thiazolidinedione.

**8.** Dispositif médical selon la revendication 7, dans lequel ladite thiazolidinedione est sélectionnée parmi le groupe constitué de la rosiglitazone, de la pioglitazone, de la troglitazone, de la darglitazone, de l'englitazone, de la ciglitazone, de dérivés de ces dernières et de combinaisons de ces dernières.

**9.** Dispositif médical selon la revendication 8, dans lequel ledit revêtement contient en outre un polymère biocompatible sélectionné parmi le groupe constitué de la polyvinylpyrrolidone, du polytétrafluoroéthylène, de l'acide poly-L-lactique, du polycaprolactone, du polyéthylèneglycol, du polystyrène, d'acrylates, de polyesters et de mélanges de ces derniers.

**10.** Dispositif médical selon la revendication 9, dans lequel ledit revêtement comprend :

entre 50 µg et 250 µg de ciglitazone et de polycaprolactone, dans lequel ladite ciglitazone et ledit polycaprolactone sont dans un rapport relatif l'une par rapport à l'autre d'approximativement 1 part de ciglitazone à approximativement de 1 à 9 parts de polycaprolactone.

**11.** Procédé de production d'un dispositif médical comprenant :

le fait de pourvoir le dispositif médical d'un revêtement ;
le mélange d'au moins une thiazolidinedione sélectionnée parmi le groupe constitué de la rosiglitazone, de la pioglitazone, de la troglitazone, de la darglitazone, de l'englitazone, de la ciglitazone, de dérivés de ces dernières et de combinaisons de ces dernières avec un composé vecteur ; et
le revêtement desdits dispositifs médicaux à l'aide de ladite thiazolidinedione mélangée audit composé vecteur.

**12.** Procédé selon la revendication 11, dans lequel ledit dispositif médical est un stent vasculaire.

**13.** Procédé selon la revendication 11, en outre, dans lequel ledit composé vecteur est un polymère biocompatible sélectionné parmi le groupe constitué de la polyvinylpyrrolidone, du polytétrafluoroéthylène, de l'acide poly-L-lactique, de la caprolactone, du polyéthylèneglycol, du polystyrène, d'acrylates, de polyesters et de mélanges de ces derniers.

**14.** Dispositif médical selon les revendications 8 à 10, comprenant un stent ayant un revêtement, le revêtement comprenant au moins une thiazolidinedione sélectionnée parmi le groupe constitué de la rosiglitazone, de la pioglitazone, de la troglitazone, de la darglitazone, de l'englitazone, de la ciglitazone, de dérivés de ces dernières et de combinaisons de ces dernières ; et

au moins un agent thérapeutique supplémentaire sélectionné parmi le groupe constitué d'agents anti-plaquettes, d'agents anti-migratoires, d'agents anti-fibrotiques, d'agents anti-proliférateurs, d'agents anti-inflammatoires et de combinaisons de ces derniers, sous réserve que ledit agent thérapeutique supplémentaire ne soit pas un agoniste PPARγ.

**15.** Dispositif médical selon la revendication 14, dans lequel ledit au moins un agent thérapeutique supplémentaire est sélectionné parmi le groupe constitué de d'oligonucléotides 'antisense', de la rapamycine, d'analogues de la

rapamycine, de l'exochéline, d'agents inhibiteurs de la n-acétylcystéine, d'agents inhibiteurs de la chaperone et de combinaisons de ces derniers.

**16.** Dispositif médical selon la revendication 15, dans lequel ledit oligonucléotide antisense est un oligonucléotide anti-c-myc.

**17.** Dispositif médical selon la revendication 15, dans lequel ledit agent inhibiteur de chaperone est la geldanamycine.

**18.** Dispositif médical selon la revendication 15, dans lequel ledit dérivé de la rapamycine est la 40-0-(2-hydroxyéthyl)-rapamycine.

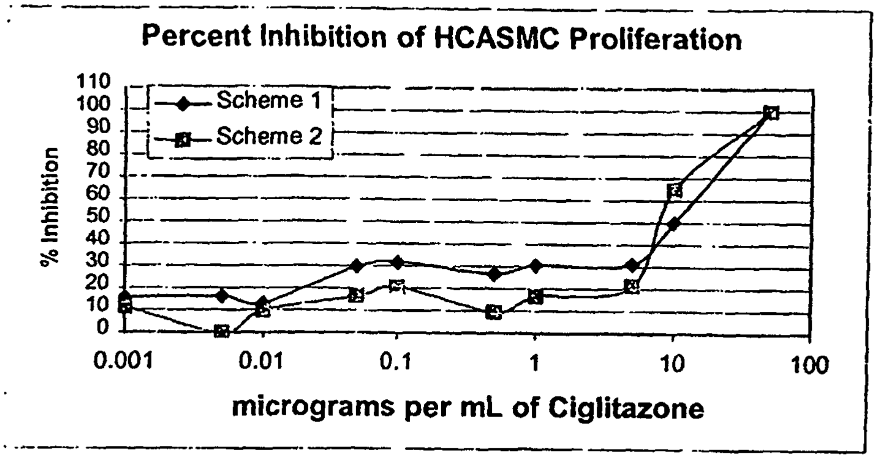


Figure 1a

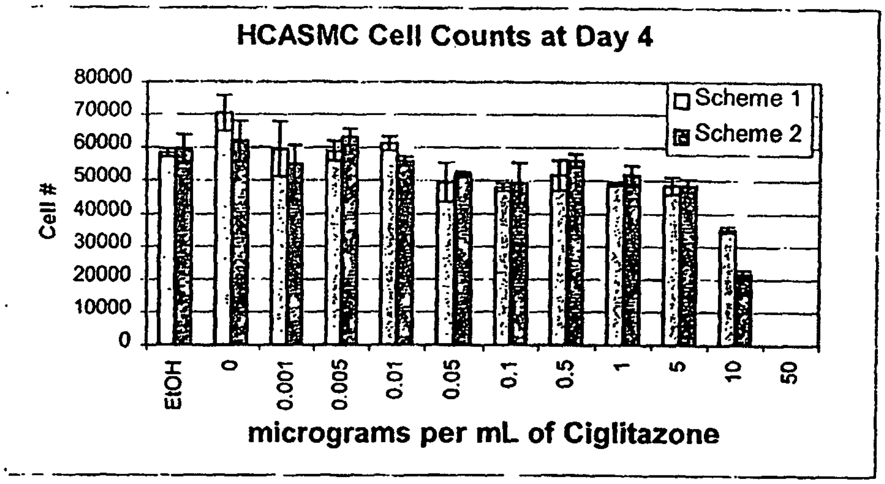


Figure 1b

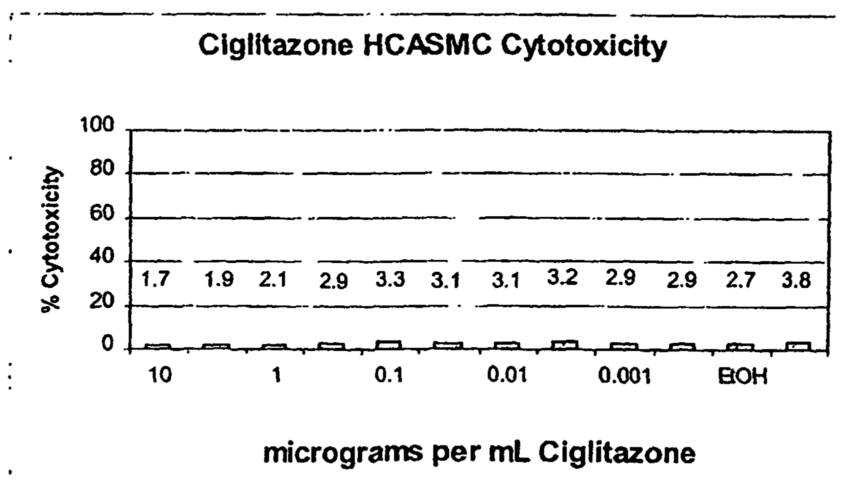


Figure 1c

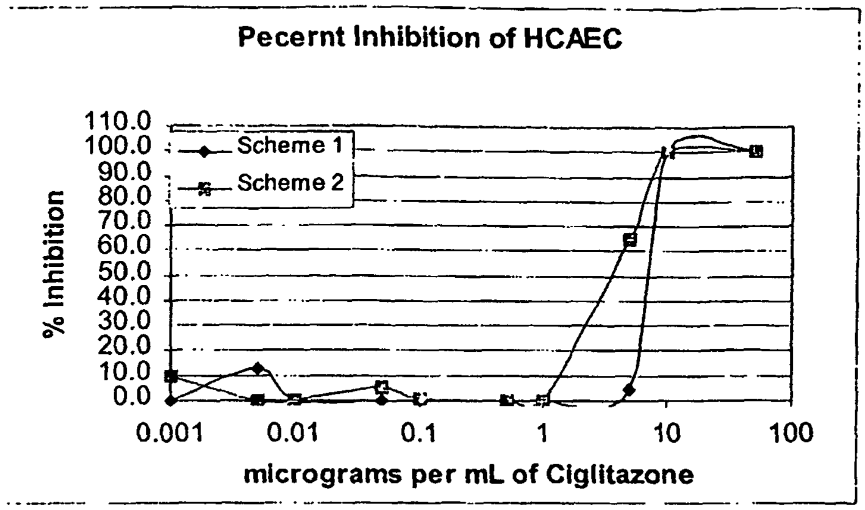


Figure 2a

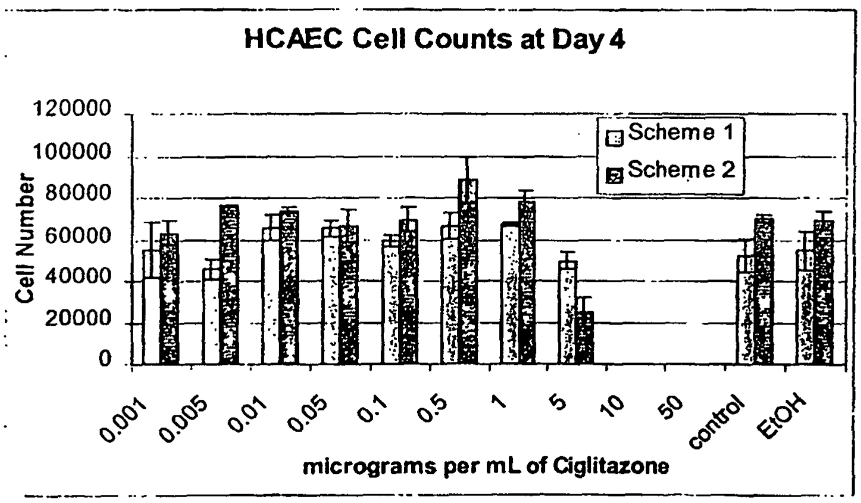


Figure 2b

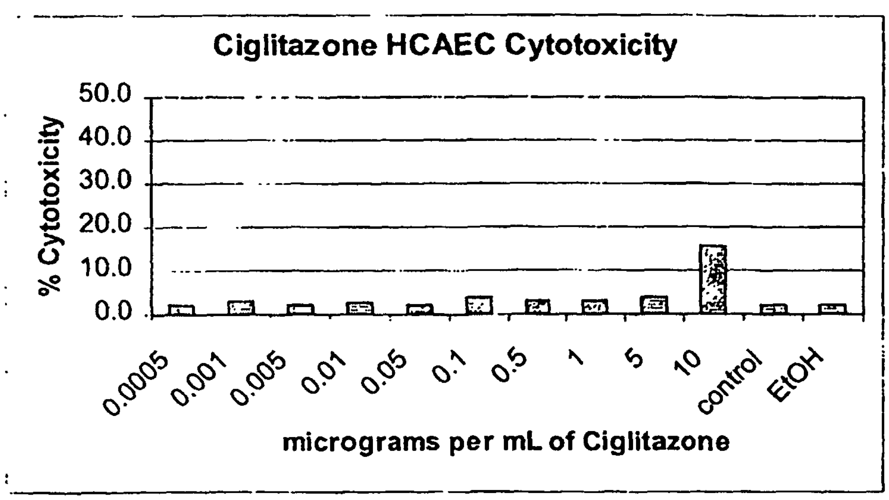


Figure 2c

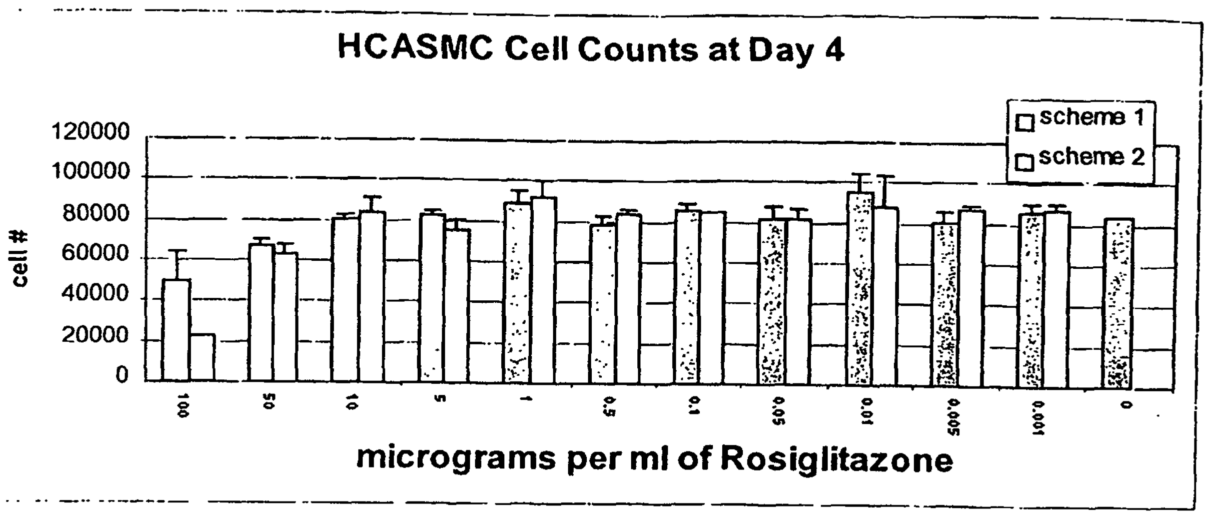
HCASMC Cell Counts at Day 4
scheme 1
scheme 2
120000
100000
80000
60000
40000
20000
0
cell #
100
50
10
5
1
0.5
0.1
0.05
0.01
0.005
0.001
0
micrograms per ml of Rosiglitazone

Figure 3